# EUROPEAN PATENT APPLICATION

(11) **EP 1 835 036 A1**
(43) Date of publication of application: **19.09.2007**
(21) Application number: 06290425.5
(22) Date of filing: 16.03.2006
(51) Int. Cl.: C12Q 1/68

(54) **Methods and compositions for the detection and treatment of cancers**

(71) Applicant: Exonhit Therapeutics SA, 75017 Paris (FR); Hopitaux Universitaires de Geneve, 1211 Geneva 14 (CH); UNIVERSITE DE GENEVE, 1211 Genève 4 (CH)
(72) Inventor: Piguet, Vincent, 1224 Chêne-Bougeries (CH); Correa Rocha, Rafael, 1006 Lausanne (CH); Bracco, Laurent, 92250 La Garenne Colombes (FR)
(74) Representative: Becker, Philippe

(57) **Abstract**

The present invention relates to methods and compositions for the detection and treatment of melanoma and skin cancers. More particularly, the invention discloses that BCSC-1 expression is altered in melanoma and skin cancer cells, allowing the design of effective detection methods and kits for such conditions. The invention also shows that restoring or increasing expression of BCSC-1 in melanoma and skin cancer cells suppresses tumorigenicity and represents a novel and effective approach for the treatment of melanoma and skin cancers. The invention may be used to detect the presence, stage or type of melanoma and skin cancers, as well as any predisposition thereto. The invention may be used in any mammalian subject, particularly human subjects.

### Annex to the application documents - subsequently filed sequences listing

SEQUENCE LISTING

<110> Exonhit Therapeutics SA
Hopitaux Universitaires de Genève
Université de Genève

<120> Detection and Treatment of Cancers

<130> B0462EP

<160> 14

<170> PatentIn version 3.3

<210> 1
<211> 26
<212> DNA
<213> Artificial sequence

<220>
<223> Primer

<400> 1
aaattatgac tgcagctgtt ttcact 26

<210> 2
<211> 21
<212> DNA
<213> Artificial sequence

<220>
<223> Primer

<400> 2
gtagaacagg ctgggcttcc t 21

<210> 3
<211> 27
<212> DNA
<213> Artificial sequence

<220>
<223> Primer

<400> 3
ctgatttact acaatgaggt gcatacc 27

<210> 4
<211> 22
<212> DNA
<213> Artificial sequence

<220>
<223> Primer

<400> 4
catcaaatga cctggcttca tg 22

<210> 5
<211> 27
<212> DNA

<213>  Artificial sequence

<220>
<223>  Primer

<400>  5
agaaacacag gtaggaagaa aatgtga                                          27


<210>  6
<211>  27
<212>  DNA
<213>  Artificial sequence

<220>
<223>  Primer

<400>  6
tcatgtagca ctagagtctg tgtgtca                                          27


<210>  7
<211>  22
<212>  DNA
<213>  Artificial sequence

<220>
<223>  Primer

<400>  7
agcctgatgt caacctcacc at                                               22


<210>  8
<211>  21
<212>  DNA
<213>  Artificial sequence

<220>
<223>  Primer

<400>  8
catgtccttg gtctggagca a                                                21


<210>  9
<211>  20
<212>  DNA
<213>  Artificial sequence

<220>
<223>  Primer

<400>  9
agcacagtcc aggctttgga                                                  20


<210>  10
<211>  21
<212>  DNA
<213>  Artificial sequence

<220>
<223>  Primer

<400>  10
caggaaccat ttgcattttg g                                                21

<210>  11
<211>  21
<212>  DNA
<213>  Artificial sequence

<220>
<223>  Primer

<400>  11
aatgggagct tctggaaagg a                                                21

<210>  12
<211>  18
<212>  DNA
<213>  Artificial sequence

<220>
<223>  Primer

<400>  12
gcatggtgga gcctgcat                                                    18

<210>  13
<211>  786
<212>  PRT
<213>  Homo sapiens

<400>  13

Met Val His Phe Cys Gly Leu Leu Thr Leu His Arg Glu Pro Val Pro
1               5                   10                  15

Leu Lys Ser Ile Ser Val Ser Val Asn Ile Tyr Glu Phe Val Ala Gly
            20                  25                  30

Val Ser Ala Thr Leu Asn Tyr Glu Asn Glu Glu Lys Val Pro Leu Glu
        35                  40                  45

Ala Phe Phe Val Phe Pro Met Asp Glu Asp Ser Ala Val Tyr Ser Phe
    50                  55                  60

Glu Ala Leu Val Asp Gly Lys Lys Ile Val Ala Glu Leu Gln Asp Lys
65                  70                  75                  80

Met Lys Ala Arg Thr Asn Tyr Glu Lys Ala Ile Ser Gln Gly His Gln
                85                  90                  95

Ala Phe Leu Leu Glu Gly Asp Ser Ser Ser Arg Asp Val Phe Ser Cys
            100                 105                 110

Asn Val Gly Asn Leu Gln Pro Gly Ser Lys Ala Ala Val Thr Leu Lys
        115                 120                 125

Tyr Val Gln Glu Leu Pro Leu Glu Ala Asp Gly Ala Leu Arg Phe Val
    130                 135                 140

Leu Pro Ala Val Leu Asn Pro Arg Tyr Gln Phe Ser Gly Ser Ser Lys
145                 150                 155                 160

Asp Ser Cys Leu Asn Val Lys Thr Pro Ile Val Pro Val Glu Asp Leu
                165                 170                 175

Pro Tyr Thr Leu Ser Met Val Ala Thr Ile Asp Ser Gln His Gly Ile
            180                 185                 190

Glu Lys Val Gln Ser Asn Cys Pro Leu Ser Pro Thr Glu Tyr Leu Gly
        195                 200                 205

Glu Asp Lys Thr Ser Ala Gln Val Ser Leu Ala Ala Gly His Lys Phe
    210                 215                 220

Asp Arg Asp Val Glu Leu Leu Ile Tyr Tyr Asn Glu Val His Thr Pro
225                 230                 235                 240

Ser Val Val Leu Glu Met Gly Met Pro Asn Met Lys Pro Gly His Leu
                245                 250                 255

Met Gly Asp Pro Ser Ala Met Val Ser Phe Tyr Pro Asn Ile Pro Glu
            260                 265                 270

Asp Gln Pro Ser Asn Thr Cys Gly Glu Phe Ile Phe Leu Met Asp Arg
        275                 280                 285

Ser Gly Ser Met Gln Ser Pro Met Ser Ser Gln Asp Thr Ser Gln Leu
    290                 295                 300

Arg Ile Gln Ala Ala Lys Glu Thr Leu Ile Leu Leu Leu Lys Ser Leu
305                 310                 315                 320

Pro Ile Gly Cys Tyr Phe Asn Ile Tyr Gly Phe Gly Ser Ser Tyr Glu
                325                 330                 335

Ala Cys Phe Pro Glu Ser Val Lys Tyr Thr Gln Gln Thr Met Glu Glu
            340                 345                 350

Ala Leu Gly Arg Val Lys Leu Met Gln Ala Asp Leu Gly Gly Thr Glu
        355                 360                 365

Ile Leu Ala Pro Leu Gln Asn Ile Tyr Arg Gly Pro Ser Ile Pro Gly
    370                 375                 380

His Pro Leu Gln Leu Phe Val Phe Thr Asp Gly Glu Val Thr Asp Thr
385                 390                 395                 400

Phe Ser Val Ile Lys Glu Val Arg Ile Asn Arg Gln Lys His Arg Cys
                405                 410                 415

Phe Ser Phe Gly Ile Gly Glu Gly Thr Ser Thr Ser Leu Ile Lys Gly
            420                 425                 430

Ile Ala Arg Ala Ser Gly Gly Thr Ser Glu Phe Ile Thr Gly Lys Asp
        435                 440                 445

Arg Met Gln Ser Lys Ala Leu Arg Thr Leu Lys Arg Ser Leu Gln Pro
    450                 455                 460

Val Val Glu Asp Val Ser Leu Ser Trp His Leu Pro Pro Gly Leu Ser
465                 470                 475                 480

Ala Lys Met Leu Ser Pro Glu Gln Thr Val Ile Phe Arg Gly Gln Arg
                485                 490                 495

Leu Ile Ser Tyr Ala Gln Leu Thr Gly Arg Met Pro Ala Ala Glu Thr
            500                 505                 510

Thr Gly Glu Val Cys Leu Lys Tyr Thr Leu Gln Gly Lys Thr Phe Glu
        515                 520                 525

Asp Lys Val Thr Phe Pro Leu Gln Pro Lys Pro Asp Val Asn Leu Thr
    530                 535                 540

Ile His Arg Leu Ala Ala Lys Ser Leu Leu Gln Thr Lys Asp Met Gly
545                 550                 555                 560

Leu Arg Glu Thr Pro Ala Ser Asp Lys Lys Asp Ala Leu Asn Leu Ser
                565                 570                 575

Leu Glu Ser Gly Val Ile Ser Ser Phe Thr Ala Phe Ile Ala Ile Asn
            580                 585                 590

Lys Glu Leu Asn Lys Pro Val Gln Gly Pro Leu Ala His Arg Asp Val
        595                 600                 605

Pro Arg Pro Ile Leu Leu Gly Ala Ser Ala Pro Leu Lys Ile Lys Cys
    610                 615                 620

Gln Ser Gly Phe Arg Lys Ala Leu His Ser Asp Arg Pro Pro Ser Ala
625                 630                 635                 640

Ser Gln Pro Arg Gly Glu Leu Met Cys Tyr Lys Ala Lys Thr Phe Gln
                645                 650                 655

Met Asp Asp Tyr Ser Leu Cys Gly Leu Ile Ser His Lys Asp Gln His
            660                 665                 670

Ser Pro Gly Phe Gly Glu Asn His Leu Val Gln Leu Ile Tyr His Gln
        675                 680                 685

Asn Ala Asn Gly Ser Trp Asp Leu Asn Glu Asp Leu Ala Lys Ile Leu
    690                 695                 700

Gly Met Ser Leu Glu Glu Ile Met Ala Ala Gln Pro Ala Glu Leu Val
705                 710                 715                 720

Asp Ser Ser Gly Trp Ala Thr Ile Leu Ala Val Ile Trp Leu His Ser
                725                 730                 735

Asn Gly Lys Asp Leu Lys Cys Glu Trp Glu Leu Leu Glu Arg Lys Ala
            740                 745                 750

Val Ala Trp Met Arg Ala His Ala Gly Ser Thr Met Pro Ser Val Val
        755                 760                 765

Lys Ala Ala Ile Thr Phe Leu Lys Ser Ser Val Asp Pro Ala Ile Phe
    770                 775                 780

Ala Phe
785

<210>  14
<211>  3438
<212>  DNA
<213>  Homo sapiens

<400>  14
cggttctttc cggaaattat gactgcagct gttttcactc cgctgtgact cagagcgctc      60

cgggctgcag gagaggaaga aatcttgcat caccatggtg cacttctgtg gcctactcac     120

cctccaccgg gagccagtgc cgctgaagag tatctctgtg agcgtgaaca tttacgagtt     180

tgtggctggt gtgtctgcaa ctttgaacta cgagaatgag gagaaagttc ctttggaggc     240

cttctttgtg ttccccatgg atgaagactc tgctgtttac agctttgagg ccttggtgga     300

tgggaagaaa attgtagcag aattacaaga caagatgaag gcccgcacca actatgagaa     360

agccatctcc cagggccacc aggccttctt attggagggg gacagcagct ccagggatgt     420

cttctcttgc aatgtgggta acctccaacc tgggtcgaag gcggcagtca ccctgaagta     480

tgtgcaggag ctgcctctgg aagcagatgg ggctctgcgc tttgtgctcc cagctgtcct     540

gaatcctaga taccagttct ctgggtcgtc taaggacagt tgccttaatg tgaagactcc     600

tatagtccct gtggaggacc tgccctacac actcagcatg gtcgccacca tagattccca 660
gcatggcatt gagaaggtcc aatccaactg ccccttgagt cctaccgagt acctaggaga 720
ggacaagact tctgctcagg tttccctggc tgctggacac aagtttgatc gggacgtgga 780
actcctgatt tactacaatg aggtgcatac ccccagcgtg gttttggaga tggggatgcc 840
taacatgaag ccaggtcatt tgatgggaga tccatctgca atggtgagtt tctatccaaa 900
tatcccagaa gatcaaccat caaatacctg tggagagttt atctttctca tggaccgctc 960
gggaagtatg cagagcccca tgagtagcca ggatacatct cagctgcgaa tacaggcagc 1020
caaggaaaca ctgattttgc tgctgaagag tttacctata ggctgttatt tcaacatcta 1080
tggatttggc tcttcctatg aggcatgctt tccggagagt gtgaagtaca ctcagcaaac 1140
aatggaggag gctctgggga gagtgaagct tatgcaggcc gacctagggg gcactgaaat 1200
cttggcacca ctccagaaca tttacagggg accctccatc ccaggccacc ccctacagct 1260
ttttgtcttt acagatggag aagttacaga cacgtttagt gtaattaaag aagttaggat 1320
caacagacag aaacacaggt gtttctcatt tggtattgga gaaggcacct ccaccagcct 1380
aataaaaggt attgcccggg catcaggggg cacctcagaa tttatcacag gcaaagacag 1440
gatgcagtcc aaggctctca ggactctgaa acgctctctg cagcctgtgg tagaggatgt 1500
ctctctgagc tggcatttgc ctcctggtct gtctgctaaa atgctttccc cagaacagac 1560
tgtcatcttt aggggtcaga gattaatcag ctatgcccag ctgaccggga ggatgccagc 1620
agcagagaca acaggagaag tatgcctcaa atatacactc cagggcaaga cttttgagga 1680
taaggtgaca tttcctctac aacccaagcc tgatgtcaac ctcaccattc accgccttgc 1740
tgccaagtcc ttgctccaga ccaaggacat gggcctcagg gagactccag caagtgataa 1800
aaaagatgca ttgaacctta gccttgagtc tggtgtcata agctccttca cagctttcat 1860
tgctatcaat aaggagctca acaagccggt tcaggggcct ctggctcata gggacgtccc 1920
aaggccaatt ctgttgggtg cttctgcccc attgaagata aaatgccaat caggttttcg 1980
aaaggcctta cactctgacc gtcctccttc tgcatctcag cccagagggg aacttatgtg 2040
ttataaggcc aagacattcc agatggacga ttacagtctc tgtgggttga taagtcacaa 2100
ggaccagcac agtccaggct ttggagagaa tcaccttgtg cagctgattt accaccaaaa 2160
tgcaaatggt tcctgggatc tgaatgaaga tctagccaag atcctaggta tgagtttgga 2220
agaaataatg gctgcacagc ctgccgagct tgtggattcc tcaggctggg ccaccatcct 2280
ggccgtgatc tggctgcaca gcaatggtaa ggacttgaag tgtgaatggg agcttctgga 2340
aaggaaggcc gtggcctgga tgcgtgccca tgcaggctcc accatgcctt cggttgtgaa 2400
agctgctatt actttcctga agtcatctgt ggatcctgct atctttgcct tttgaagata 2460
ccatccagaa aaagaagtgc ctttaatttg ctactgtcat ttcctctagt atcacttttg 2520

ctgtgatgat gtgttcttgt gtattataac tctttatttt ttgccataaa agtaaaggat 2580
gcttactcca cttcgcttct ctgctccagg ttcactttgg atatgatctt tcttttccca 2640
acatatgccc tcagaaaagt gacagtggtc ccagaaccta ttccctttct tgagggagtt 2700
caaaacattc ataggcagta atgttcctcc cagggtttcc agggaaacaa catgaaaaac 2760
aggtgacatg aactacagac taaagattgc agcatttatg ttagagaatg cttgaattag 2820
agaattttct gcattatctt tgtctgttca ctttctatct tatatactta tcagggccat 2880
actggtaagc ttgcgtagga ggagttagag ggaagttgaa agccaacatc tggatcaatg 2940
taatgtcaag atcacaaaga cagagactgc aggggtccac tgtgagaggt gacactgttg 3000
gggaccttcc tgattcattc ttcttgggct ttgctagcct gtacaaccta catgtctttt 3060
cttccactgc ctgaaagact tgggttgaac tataactgtt ggagagagat gttcctcttt 3120
aatcatgaaa caccttaaga agtctataat gcaatcctta gtcctaccct gaacctatgt 3180
gtcctctaag tcaggccctg atctagtgca gtaaagggaa gggtgggctt aatgggagct 3240
ttgcctggga cctgaacctg gagcacttac cgcattagga agaaaggagc tccccgtaat 3300
cgttcctgac ccttgtgtct catataccct atcctggtgg aaatgaccct atttgatatg 3360
ctgtccctta aaataacttg tatcaatatt aaaatgacta tttctaccct ttgatgagta 3420
aaaaaaaaaa aaaaaaaa 3438

## Description

The present invention relates to methods and compositions for the detection and treatment of melanoma and skin cancers. More particularly, the invention discloses that BCSC-1 expression is altered in melanoma and skin cancer cells, allowing the design of effective detection methods and kits for such conditions. The invention also shows that restoring or increasing expression of BCSC-1 in melanoma and skin cancer cells suppresses tumorigenicity and represents a novel and effective approach for the treatment of melanoma and skin cancers. The invention may be used to detect the presence, stage or type of melanoma and skin cancers, as well as any predisposition thereto. The invention may be used in any mammalian subject, particularly human subjects.

Melanoma is an aggressive cancer with a propensity to cause widespread metastatic disease. Diagnosis is essential at an early stage since to this day the only successful treatment is the excision of the tumor when still confined to the skin. Unfortunately, melanoma is largely resistant to conventional chemotherapy and new treatments are urgently needed for metastatic melanoma.

In order to study the gene expression profiling of pigmented skin lesions and melanoma, global analysis of alternative splicing of benign nevus and metastatic melanomas was studied using the DATAS technology. A DATAS library for benign naevus-metastatic melanoma including 217 differentially expressed clones between benign nevus and metastatic melanomas was generated with this method. Several clones were screened by Real-Time PCR, that could be implicated in melanoma progression based on their implication in apoptosis, cell cycle, metabolism, etc. Among these genes, we identified that BCSC-1 (Martin et.al. PNAS 2003) was consistently down-regulated in patients with metastatic melanoma.

We further investigated the expression of BCSC-1 by Real-Time PCR in a cohort of 70 skin biopsies from patients with benign nevus, atypical nevus, primary melanoma or metastatic melanoma. The results showed that BCSC-1 expression was decreased in all melanoma patients in comparison with healthy donors. These results were confirmed in melanoma cell lines and in all the melanoma patients analyzed.

Importantly, when BCSC-1 gene was expressed ectopically in melanoma cell lines, melanoma cells stopped proliferating and underwent apoptosis. The expression of the BCSC protein in these cells was confirmed by Western Blot.

In conclusion, the present invention shows that BCSC-1 is down-regulated in a skin cancer and that induction of BCSC-1 expression in said cancer cells resulted in an inhibition of tumor progression. This data show that BCSC-1 plays a crucial role in the pathogenesis of melanoma and skin cancer, and represents a novel prognosis marker as well as a valuable target for drug development for melanoma and skin cancers.

A first object of this invention resides in methods for detecting the presence, stage or type of a melanoma or skin cancer in a subject, the method comprising detecting in vitro or ex vivo the presence of an altered BCSC-1 gene expression in a sample from the subject, the presence of such an altered BCSC-1 gene expression being indicative of the presence, stage or type of a melanoma or skin cancer in said subject.

According to specific embodiments of the method, the altered expression is a reduced expression of a BCSC-1 gene in said sample, even more preferably a reduced expression of (a) long isoform(s) of a BCSC-1 gene in said sample.

An other object of this invention resides in methods for detecting the presence, stage or type of a cancer in a subject, the method comprising determining in vitro or ex vivo the (relative) amount of (a) long isoform(s) of BCSC-1 gene or protein in a sample from the subject, such amount being indicative of the presence, stage or type of a cancer in said subject. In a particular embodiment, the amount determined is compared to a control or mean value, or to that measured in a control sample. In an other embodiment, the ratio BCSC-1 long isoform(s) / BCSC-1 short isoform(s) is determined, and any decrease in such ratio is indicative of the presence of such a cancer. Such a method may be used to detect any cancer particularly solid cancers.

In this regard, an other object of this invention resides in methods for detecting the presence, stage or type of a cancer in a subject, the method comprising determining in vitro or ex vivo the ratio BCSC-1 long isoform(s) / BCSC-1 short isoform(s), a decrease in such ratio as compared to control condition or reference or mean value is indicative of the presence of such a cancer. Such a method may be used to detect any cancer particularly solid cancers.

An other object of this invention resides in methods for detecting the presence, stage or type of a cancer in a subject, the method comprising .determining in vitro or ex vivo the (relative) amount of BCSC-1 protein in a fluid sample derived from the subject, such amount being indicative of the presence, stage or type of a cancer in said subject. Typically, (relative) amount of long isoform(s) of the BCSC-1 protein are determined, a decrease in such amounts as compared to control or reference value being indicative of the presence of a cancer. In a typical embodiment, the fluid sample derives (e.g., by dilution, concentration, purification, separation, etc.) from total blood, serum, plasma, urine, etc. Such a method maybe used to detect any cancer particularly solid cancers.

A further object of this invention is a method of assessing the efficacy of a treatment of melanoma and skin cancer in a subject, the method comprising comparing (in vitro or ex vivo) BCSC-1 gene expression in a sample from the subject prior to and after said treatment, an increased expression being indicative of a positive response to treatment.

The invention also relates to the use of a BCSC-1 protein or of a nucleic acid encoding a BCSC-1 protein for the manufacture of a medicament for treating melanoma or a skin cancer, as well as to a corresponding method of treatment.

The invention also concerns the use of a BCSC-1 agonist for the manufacture of a. pharmaceutical composition for treating melanoma or skin cancer, as well as corresponding method of treatment.

A further aspect of this invention resides in the use of a compound that stimulates BCSC-1 gene expression for the manufacture of a pharmaceutical composition for treating melanoma or skin cancer, as well as in a corresponding method of treatment.

The invention also resides in methods of treating melanoma or skin cancer in a subject in need thereof, comprising administering to said subject an effective amount of a compound as defined above. In a preferred embodiment, the subject has an altered BCSC-1 gene expression.

The invention further relates to methods of selecting biologically active compounds on melanoma or skin cancer, the method comprising a step of selecting compounds that mimic or stimulate BCSC-1 expression or activity.

In a specific embodiment, the method comprises contacting a test compound with a recombinant host cell comprising a reporter construct, said reporter construct comprising a reporter gene under the control of a BCSC-1 gene promoter, and selecting the test compounds that stimulate expression of the reporter gene.

The skin cancer may be selected from basal cell carcinoma, squamous cell carcinoma, Merkel carcinoma, primary cutaneous lymphoma, as well as from any other cell proliferative disease of the skin or related surface tissues.

BCSC-1 gene expression may be detected by any technique known per se in the art, such as by sequencing, selective hybridization, selective amplification and/or specific ligand binding. In a particular embodiment, the invention uses reagents and/or techniques allowing the detection of long isoforms of BCSC-1, or the discrimination between long and short isoforms, or the measure of the ratio long isoform(s) / short isoform(s).

A further aspect of this invention resides in a nucleic acid primer that allows (specific) amplification of (a) long isoform(s) of BCSC-1, or allows to discriminate between long and short isoforms.

A further aspect of this invention resides in a nucleic acid probe that (specifically) hybridizes to a long isoform of BCSC-1, or allows to discriminate between long and short isoforms.

A further aspect of this invention resides in an antibody (including derivatives thereof and producing hybridomas), that (specifically) binds a long isoform of a BCSC-1 protein or allows to discriminate between long and short isoforms.

The invention further relates to kits comprising a primer, probe or antibody as defined above. Such kits may comprise a container or support, and/or reagents to perform an amplification, hybridization or binding reaction.

A particular aspect of this invention resides in compositions of matter comprising primers, probes, and/or antibodies, which are designed to specifically detect at least one long isoform of BCSC-1.

### LEGEND TO THE FIGURES

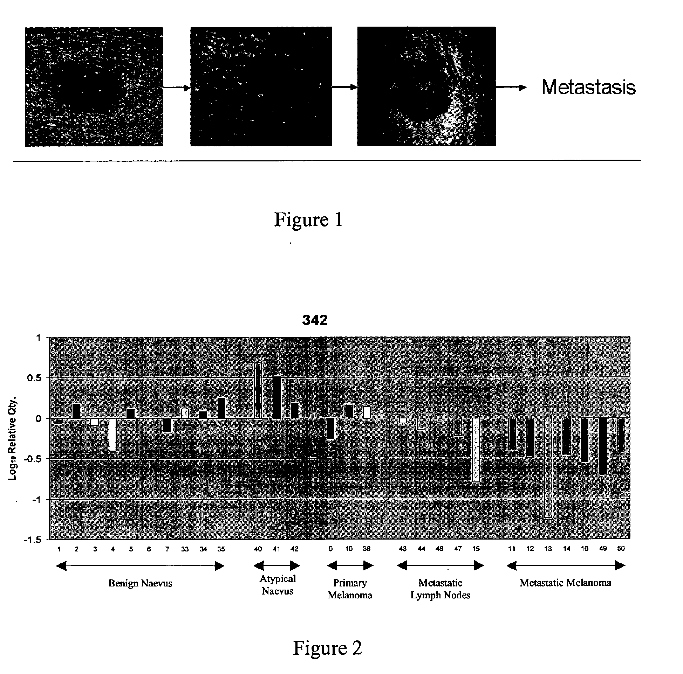

Figure 1 : Progression model of pigmented skin lesions
Figure 2 : BCSC-1 expression is down-regulated in melanoma patients. RNA expression by Real-time PCR of BCSC-1 gene in skin biopsies from patients with benign nevus or melanoma skin lesions. The zero value represents the mean expression for this gene in the patients with benign nevus (healthy donors). All patients with metastatic melanoma show a lower expression (up to 20 fold lower) in comparison with mean expression values in healthy donors.

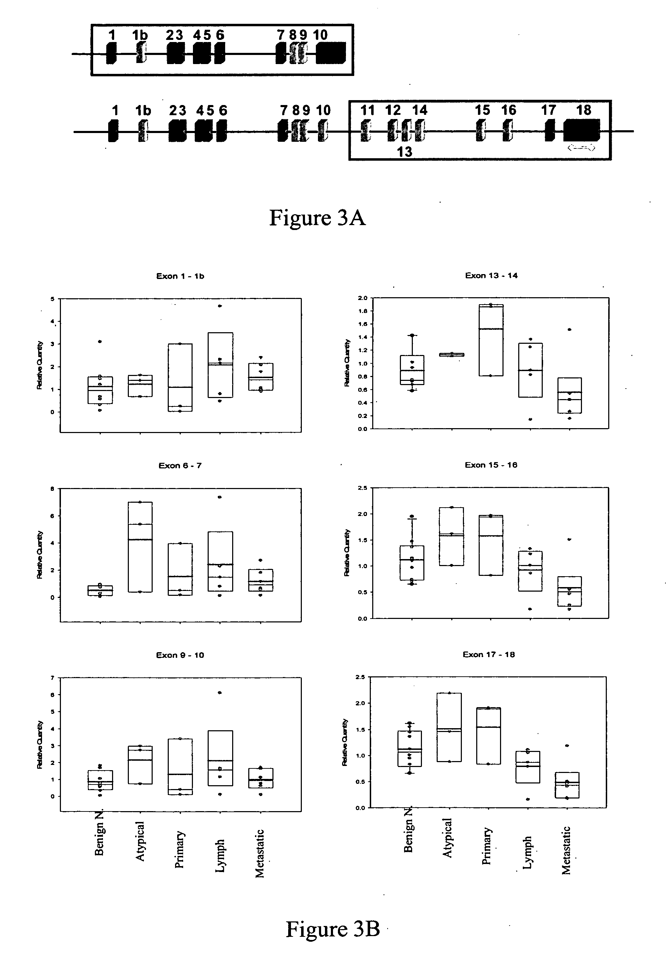

Figure 3 : Long isoforms of BCSC-1 are selectively down-regulated in melanoma patients. Several isoforms for BCSC-1 gene have been described. Real-Time PCR analysis of different exons shows that exons present exclusively in long isoforms (Exons 13-18) are the exons decreased in melanoma patients.

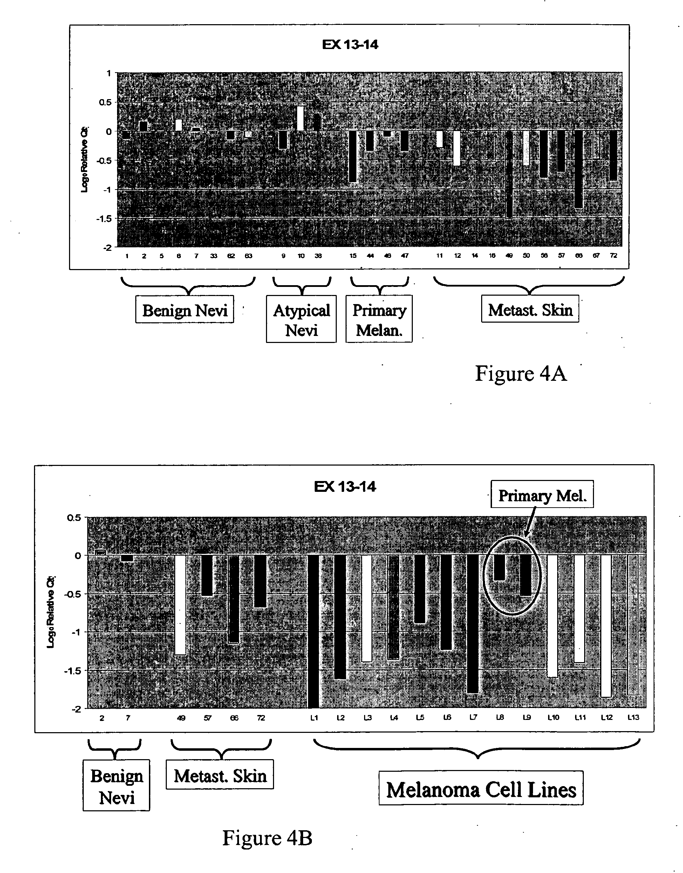

Figure 4 : BCSC-1 expression is down-regulated in melanoma patients and melanoma cell lines. The results were confirmed in a larger cohort of patients and also in melanoma cell lines. The expression of longer isoforms of the BCSC-1 gene is markedly decreased in melanoma patients and melanoma cell lines.

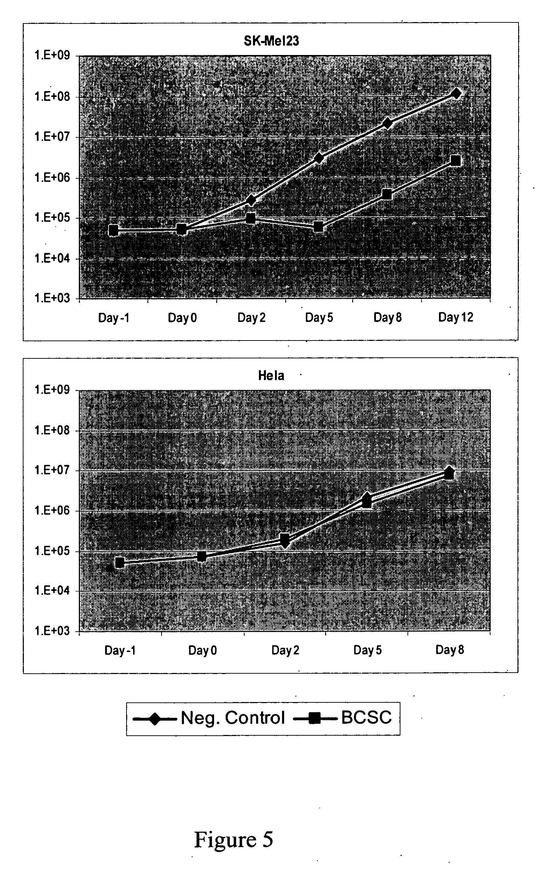

Figure 5 : Ectopic Expression of BCSC-1 decreases melanoma cells proliferation. Melanoma cell lines were transduced with a lentivector encoding BCSC-1. Melanoma cells expressing BCSC-1 stop or decrease markedly their proliferation in comparison with negative controls. This effect is not observed in non-melanoma cell lines (Hela) also transduced with the BCSC vector.

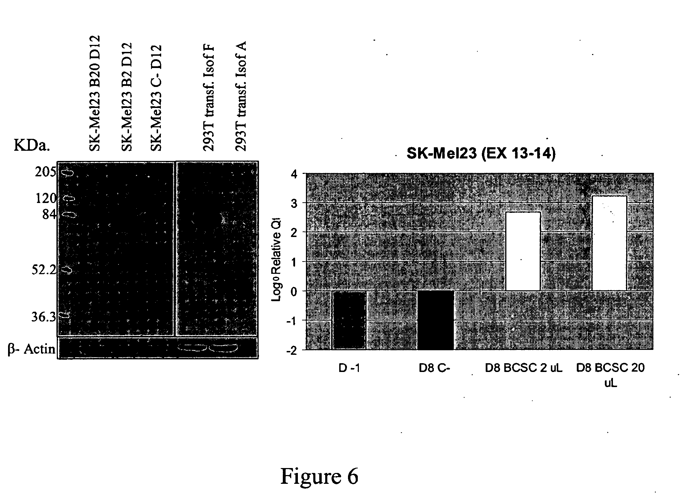

Figure 6 : Ectopic Expression of BCSC-1 is confirmed by Western Blot and Real-time PCR. (Left) The production of the BCSC-1 protein is confirmed by Western Blot using an anti-HA antibody in SK-Mel23 cells transduced with increasing doses (2 and 20 µl) of BCSC-1 encoding lentiviral vector at day 12, but not in the cells transduced with an empty vector (C-). Transfected 293T cells with both vector of long and short isoforms of BCSC-1 were used as positive controls also detecting BCSC-1 with an anti-HA antibody. (Right) Real-time PCR showed that SK-Mel23 cells do not express detectable levels of BCSC-1 prior to the transduction, and at day 8 when they are transduced with the empty vector. However, SK-Mel23 cells transduced with the BCSC-1 vector express high levels of BCSC-1 mRNA.

### DETAILED DESCRIPTION OF THE INVENTION

The incidence of melanoma is on the increase. It is an aggressive cancer with a propensity to cause widespread disease. New methods based on the study of global gene expression are needed in order to understand the events underlying melanoma carcinogenesis.

As disclosed on Figure 1, the transitions between benign naevus (left), dysplastic naevus (center) and malignant melanoma (right) can be analyzed using morphology techniques. There is however only a partial understanding of the molecular events that govern these transitions.

In order to identify the molecular events governing the transition between benign nevus and melanoma we have chosen to study alternative splicings in pigmented melanoma using a gene profiling technology called DATAS (Differential Analysis of Transcripts with Alternative Splicing). Through this global gene expression analysis, we have derived novel sequences resulting from tumor-associated splicing events. Using real-time semi-quantitative RT-PCR of RNA extracted from biopsies of a total of 70 patients, including biopsies of benign naevus (negative control) and different stages of pigmented skin lesions we could demonstrate that BCSC-1 is selectively down-regulated from patients with metastatic disease.

Furthermore, we could identify that the long isoforms of BCSC-1 are selectively down-regulated in melanoma, while the short isoforms are not substantially affected.

Moreover, ectopic expression from lentiviral vectors encoding BCSC-1 tagged with HA in melanoma cell lines induced a strong decrease in the proliferation of these cells.

Together, our results identify a novel gene (BCSC-1) selectively down-regulated in melanoma and skin cancers. Only selected "long" isoforms of this gene (c,f) were down-regulated in metastatic melanoma, while "short" isoforms appeared not affected; opening the possibility to develop more specific diagnostic tools to detect the missing isoforms in metastatic tumors. Finally ectopic expression slowed down cell proliferation of metastatic melanoma cell lines suggesting that BCSC-1 has a functional role in metastatic disease progression.

The present invention thus proposes to use BCSC-1 gene and corresponding expression products for the diagnosis and treatment of melanoma and skin cancers, as well as for the screening of therapeutically active drugs. The invention may be used in various subjects, particularly human, including adults and children.

### DEFINITIONS

Within the context of this invention, the term "BCSC-1 gene" designates all BCSC-1 nucleotide sequences in a cell or organism, including coding sequences, non-coding sequences, regulatory sequences controlling transcription and/or translation (e.g., promoter, enhancer, terminator, etc.), as well as all corresponding expression products, such as RNAs (e.g., mRNAs). The term "gene" shall be construed to include any type of coding nucleic acid, including genomic DNA (gDNA), complementary DNA (cDNA), synthetic or semisynthetic DNA, as well as any form of corresponding RNA. The term gene particularly includes recombinant nucleic acids, i.e., any non naturally occurring nucleic acid molecule created artificially, e.g., by assembling, cutting, ligating or amplifying sequences. A gene is typically double-stranded, although other forms may be contemplated, such as single-stranded. Genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof.

BCSC-1 gene sequences may be found on gene banks, such as NM_014622 and NM_198315. See also SEQ ID NO: 14, where exon boundaries are in bold character, and the start and stop codons are underlined.

The term "BCSC-1 gene" includes any variant, fragment or analog of any coding sequence as identified above. Such variants include, for instance, naturally-occurring variants due to allelic variations between individuals (e.g., polymorphisms), mutated alleles, alternative splicing forms, etc. The term variant also includes BCSC-1 gene sequences from other sources or organisms. Variants are preferably substantially homologous to with coding sequences as identified above, i.e., exhibit a nucleotide sequence identity of at least about 65%, typically at least about 75%, preferably at least about 85%, more preferably at least about 95% with coding sequence as identified above. Variants and analogs of a BCSC-1 gene typically include nucleic acid sequences which hybridize to a sequence as defined above (or a complementary strand thereof) under stringent hybridization conditions.

Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

A fragment of a BCSC-1 gene designates any portion of at least about 8 consecutive nucleotides of a sequence as disclosed above, preferably at least about 15, more preferably at least about 20 nucleotides, further preferably of at least 30 nucleotides. Fragments include all possible nucleotide lengths between 8 and 100 nucleotides, preferably between 15 and 100, more preferably between 20 and 100.

Several isoforms of the BCSC-1 gene exist, which result from alternative splicings. In this regard, long isoforms and short isoforms of BCSC-1 may be defined, depending on the (number of) exons they retain. More specifically, BCSC-1 genomic DNA comprises 18 exons, which may be alternatively spliced. The position of each exons is indicated on SEQ ID NO: 14, and is summarized in the following table, where positions are given by reference to the cDNA sequence of SEQ ID NO: 14.

| Exon | Nucleotide position |
|---|---|
| 1 | 1-79 |
| 2 | 80-137 |
| 3 | 138-340 |
| 4 | 341-563 |
| 5 | 564-739 |
| 6 | 740-854 |
| 7 | 855-1024 |
| 8 | 1025-1113 |
| 9 | 1114-1258 |
| 10 | 1259-1338 |
| 11 | 1339-1453 |
| 12 | 1454-1618 |
| 13 | 1619-1719 |
| 14 | 1720-1973 |
| 15 | 1974-2117 |
| 16 | 2118-2248 |
| 17 | 2249-2375 |
| 18 | 2376-3419 |

Within the context of the present invention, a long BCSC-1 isoform is an isoform comprising the nucleotide sequence or corresponding amino acid sequence of at least one of exons 13-18. A short BCSC-1 isoform is an isoform which does not comprise the nucleotide sequence or corresponding amino acid sequence of any one of exons 13-18. Examples of long BCSC-1 isoforms comprise the nucleotide sequence or corresponding amino acid sequence of exons 1-13, 1-14, 1-15, 1-16, 1-17 or 1-18. In this regard, a long isoform is depicted in NM_014622, which contains all of exons 11-18. Similarly, isoforms depicted in AY366504 and AY366507, which contain a deletion of exons 8-16 but contains exon 18, are long isoforms within the context of this invention. An other example of a long isoform is disclosed in AY366508.

A BCSC-1 protein designates any protein or polypeptide encoded by a BCSC-1 gene as disclosed above. The term "polypeptide" refers to any molecule comprising a stretch of amino acids. This term includes molecules of various lengths, such as peptides and proteins. The polypeptide may be modified, such as by glycosylations and/or acetylations and/or chemical reaction or coupling, and may contain one or several non-natural or synthetic amino acids. A specific example of a BCSC-1 polypeptide comprises all or part of the sequence depicted in NM_014622 or NM 198315 (see also SEQ ID NO: 13).

### DETECTION / DIAGNOSIS

The invention now provides diagnostic methods based on a monitoring of the BCSC-1 gene isoforms in a subject. Within the context of the present invention, the term 'diagnosis" includes the detection, monitoring, dosing, comparison, etc., at various stages, including early, pre-symptomatic stages, and late stages, in adults or children. Diagnosis typically includes the prognosis, the assessment of a predisposition or risk of development, the characterization of a subject to define most appropriate treatment (pharmacogenetics), etc.

A general object of this invention resides in a method for detecting the presence, stage or type of a cancer in a subject, the method comprising detecting in vitro or ex vivo the presence of an altered long isoform BCSC-1 gene expression in a sample from the subject, the presence of such an altered long isoform BCSC-1 gene expression being indicative of the presence, stage or type of a cancer in said subject.

A further general aspect of this invention is a method of detecting cancer, particularly solid cancer, in a subject, comprising detecting BCSC-1 long isoform(s) in a sample from the subject, a reduced expression thereof being indicative of the presence, stage or type of cancer.

A particular object of this invention resides in a method for detecting the presence, stage or type of melanoma or a skin cancer in a subject, the method comprising detecting in vitro or ex vivo the presence of an altered BCSC-1 gene expression in a sample from the subject, the presence of such an altered BCSC-1 gene expression being indicative of the presence, stage or type of a melanoma or skin cancer in said subject.

A further object of this invention is a method as described above comprising a first step of providing a sample from the subject.

Diagnostics, which analyse and predict response to a treatment or drug, or side effects to a treatment or drug, may be used to determine whether an individual should be treated with a particular treatment drug. For example, if the diagnostic indicates a likelihood that an individual will respond positively to treatment with a particular drug, the drug may be administered to the individual. Conversely, if the diagnostic indicates that an individual is likely to respond negatively to treatment with a particular drug, an alternative course of treatment may be prescribed. A negative response may be defined as either the absence of an efficacious response or the presence of toxic side effects.

The altered BCSC-1 expression may be determined by any technique known per se in the art, e.g., at the level of the RNA or polypeptide.

As disclosed in the present invention, BCSC-1 expression is altered in melanoma or skin cancer cells. More particularly, the expression level of BCSC-1 is reduced in melanoma or skin cancer cells, as compared to non-cancerous skin cells, or to mean values. Even more specifically, the expression level of long BCSC-1 isoform(s) is reduced in cancer cells, as compared to non-cancerous cells, or to mean values. Accordingly, in a preferred embodiment, the methods of the present invention comprise a step of detecting a decrease in the expression level of the BCSC-1 gene, particularly of long isoform(s) thereof. In a typical embodiment, the methods comprise a step of determining the (relative) amounts of BCSC-1 RNA or polypeptide species in the sample, particularly of BCSC-1 RNA or polypeptide long isoform(s) in the sample, and comparing said amount to a reference or mean value, or to that measured in a control tissue (e.g., non-cancerous cell), wherein a lower value is indicative of cancer. In a further particular embodiment, the method comprises determining the ratio of a long BCSC-1 RNA or protein isoform / a short BCSC-1 RNA or protein isoform, respectively, any variation in such ratio as compared to control/reference values or samples being indicative of the presence of cancer. Although the (relative) amount of several long isoforms may be determined, it is sufficient to measure that of only one long isoform, or to determine the ratio of one long isoform / one short isoform. Such a measure may be based, for instance, on a detection of exon 13, 14, 15, 16, 17 or 18 using corresponding probes or primers, or of the encoded amino acid residues using specific ligands thereof, as will be disclosed below.

Furthermore, the invention also encompasses the detection of cancer-related structural alterations within the BCSC-1 gene or polypeptide. Such alterations in the BCSC-1 gene or polypeptide may be any form of mutation(s), deletion(s), rearrangement(s) and/or insertions in the coding and/or non-coding region, alone or in various combination(s). Mutations more specifically include point mutations. Deletions may encompass any region of two or more residues in a coding or non-coding portion of the gene, such as from two residues up to the entire gene. Typical deletions affect smaller regions, such as domains (introns) or repeated sequences or fragments of less than about 50 consecutive base pairs, although larger deletions may occur as well. Insertions may encompass the addition of one or several residues in a coding or non-coding portion of the gene. Insertions may typically comprise an addition of between 1 and 50 base pairs in the gene. Rearrangement includes inversion of sequences. Structural alteration(s) of BCSC-1 may result in the creation of stop codons, frameshift mutations, amino acid substitutions, particular RNA splicing or processing, product instability, truncated polypeptide production, etc. The alteration may result in the production of a BCSC-1 polypeptide with altered function, stability, targeting or structure.

Various techniques known in the art may be used to detect or quantify altered BCSC-1 expression, including sequencing, hybridisation, amplification and/or binding to specific ligands (such as antibodies). Other suitable methods include allele-specific oligonucleotide (ASO), allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, heteroduplex analysis, RNase protection, chemical mismatch cleavage, ELISA, radio-immunoassays (RIA) and immuno-enzymatic assays (IEMA).

Amplification may be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the BCSC-1 gene or RNA are complementary to and specifically hybridize with a portion of the BCSC-1 gene or RNA. Primers that can be used to all or a portion of the BCSC-1 gene or RNA may be designed based on the sequence of BCSC-1 itself.

Most preferred primers for use in the present invention allow the amplification of (a) long isoform(s) of BCSC-1, i.e., contain a sequence that is complementary and specifically hybridizes to a sequence located within any one of exons 13-18 of the BCSC-1 gene or RNA.

In this respect, the invention also relates to a nucleic acid primer, said primer being complementary to and hybridizing specifically to a sequence located within any one of exons 13-18 of the BCSC-1 gene or RNA. The use of such primers allows the detection of long isoforms of the BCSC-1 gene or RNA in a sample.

Typical primers of this invention are single-stranded nucleic acid molecules of about 5 to 60 nucleotides in length, more preferably of about 8 to about 25 nucleotides in length. The sequence can be derived directly from the sequence of the BCSC-1 gene. Perfect complementarity is preferred, to ensure high specificity. However, certain mismatch may be tolerated.

Specific examples of such primers are disclosed in Table 1 in the experimental section (SEQ ID NO: 1-12).

The invention also concerns the use of a nucleic acid primer or a pair of nucleic acid primers as described above in a method of detecting the presence of or predisposition to cancer in a subject or in a method of assessing the response of a subject to a treatment of cancer.

In another embodiment, detection is carried out by a technique using selective hybridization.

A particular detection technique involves the use of a nucleic acid probe specific for BCSC-1 gene or RNA, followed by the detection of the presence and/or (relative) amount of a hybrid. The probe may be in suspension or immobilized on a substrate or support (as in nucleic acid array or chips technologies). The probe is typically labeled to facilitate detection of hybrids.

In this regard, a particular embodiment of this invention comprises contacting the sample from the subject with a nucleic acid probe specific for (a) long isoform(s) of BCSC-1, and assessing the formation of a hybrid. In a particular embodiment, the method comprises contacting simultaneously the sample with a set of probes that are specific, respectively, for various long isoforms of BCSC-1.

Within the context of this invention, a probe refers to a polynucleotide sequence which is complementary to and capable of specific hybridisation with a (target portion of a) BCSC-1 gene or RNA, and which is suitable for detecting the presence or (relative) amount thereof in a sample. Probes are preferably perfectly complementary to a sequence of the BCSC-1 gene or RNA. Probes typically comprise single-stranded nucleic acids of between 8 to 1000 nucleotides in length, for instance of between 10 and 800, more preferably of between 15 and 700, typically of between 20 and 500. It should be understood that longer probes may be used as well. A preferred probe of this invention is a single stranded nucleic acid molecule of between 8 to 500 nucleotides in length, which can specifically hybridize to a region contained within any one of exons 13-18 of the BCSC-1 gene or RNA.

Specificity indicates that hybridisation to the target sequence generates a specific signal which can be distinguished from the signal generated through non-specific hybridisation. Perfectly complementary sequences are preferred to design probes according to this invention. It should be understood, however, that certain a certain degree of mismatch may be tolerated, as long as the specific signal may be distinguished from non-specific hybridisation.

The sequence of the probes can be derived from the sequences of the BCSC-1 gene and RNA as provided in the present application. Nucleotide substitutions may be performed, as well as chemical modifications of the probe. Such chemical modifications may be accomplished to increase the stability of hybrids (e.g., intercalating groups) or to label the probe. Typical examples of labels include, without limitation, radioactivity, fluorescence, luminescence, enzymatic labeling, etc.

The invention also concerns the use of a nucleic acid probe as described above in a method of detecting the presence, type or stage of progression of a cancer in a subject, or in a method of assessing the response of a subject to a cancer treatment.

The invention also relates to a nucleic acid chip comprising a probe as defined above. Such chips may be produced in situ or by depositing clones, by technique known in the art, and typically comprise an array of nucleic acids displayed on a matrix, such as a (glass, polymer, metal, etc.) slide.

As indicated above, alteration in the BCSC-1 gene expression may also be detected by detecting the presence or (relative) amount of a BCSC-1 polypeptide. In this regard, a specific embodiment of this invention comprises contacting the sample (which may comprise biological fluids such as blood, plasma, serum, etc.) with a ligand specific for a BCSC-1 polypeptide, and determining the formation of a complex.

Different types of ligands may be used, such as specific antibodies. In a specific embodiment, the sample is contacted with an antibody specific for a BCSC-1 polypeptide, and the formation of an immune complex is determined. Various methods for detecting an immune complex can be used, such as ELISA, radioimmunoassays (RIA) and immuno-enzymatic assays (IEMA).

Within the context of this invention, an antibody designates a polyclonal antibody, a monoclonal antibody, as well as any fragments or derivatives thereof having substantially the same antigen specificity. Fragments include Fab, Fab'2, CDR regions, etc. Derivatives include single-chain antibodies, humanized antibodies, poly-functional antibodies, etc.

Most preferred ligands are specific for (a) long isoform(s) of BCSC-1, i.e., bind an epitope contained within amino acid sequence encodes by any one of exons 13-18.

An antibody specific for a BCSC-1 polypeptide designates an antibody that selectively binds a BCSC-1 polypeptide. More particularly, it designates an antibody raised against a BCSC-1 polypeptide or an epitope-containing fragment thereof. Although non-specific binding towards other antigens may occur, binding to the target BCSC-1 polypeptide occurs with a higher affinity and can be reliably discriminated from non-specific binding.

In a specific embodiment, the method comprises contacting a sample from the subject with (a support coated with) an antibody specific for a BCSC-1 polypeptide, and determining the presence of an immune complex. In a particular embodiment, the sample may be contacted simultaneously, or in parallel, or sequentially, with various (supports coated with) antibodies specific for different BCSC-1 isoforms.

In an other specific embodiment, the invention relates to a method of detecting the presence of or predisposition to cancer in a subject, comprising contacting in vitro or ex vivo a sample (which may comprise biological fluids such as blood, plasma, serum, etc.) from the subject with a ligand specific for a long isoform of a BCSC-1 polypeptide, and determining the (relative) amount of complexes formed, such amount being indicative of the presence of or predisposition to cancer.

The invention also concerns the use of a ligand, preferably an antibody, a fragment or a derivative thereof as described above, in a method of detecting the presence, stage or type of a cancer in a subject, or in a method of assessing the response of a subject to a cancer treatment.

The invention also relates to a diagnostic kit comprising products and reagents for detecting in a sample from a subject the presence or (relative) amount of a BCSC-1 gene, RNA or polypeptide. Said diagnostic kit according to the present invention comprises any primer, any pair of primers, any nucleic acid probe and/or any ligand, preferably antibody, described in the present invention. Said diagnostic kit according to the present invention can further comprise reagents and/or protocols for performing a hybridization, amplification or antigen-antibody immune reaction.

The diagnostic methods of the present invention can be performed in vitro, ex vivo or in vivo, preferably in vitro or ex vivo. They use a sample from the subject, to assess any alteration in BCSC-1 gene expression. The sample may be any biological sample derived from a subject, which contains nucleic acids or polypeptides. Examples of such samples include fluids, tissues, cell samples, organs, biopsies, etc. Most preferred samples are tissue biopsies, e.g., skin cancer cells, tissues or samples. The sample may be collected according to conventional techniques and used directly for diagnosis or stored. The sample may be treated prior to performing the method, in order to ensure or improve availability of nucleic acids or polypeptides for testing. Treatments include, for instance, lysis (e.g., mechanical, physical, chemical, etc.), centrifugation, etc. Also, the nucleic acids and/or polypeptides may be pre-purified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

As indicated, the sample is preferably contacted with reagents such as probes, primers or ligands in order to assess the presence and/or (relative) amount of a BCSC-1 gene or RNA or polypeptide. Contacting may be performed in any suitable device, such as a plate, tube, well, glass, etc. In specific embodiments, the contacting is performed on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

The finding of an altered BCSC-1 expression in the sample is indicative of the presence, type or stage of progression of melanoma or skin cancer. The determination of the presence of an altered BCSC-1 expression also allows the design of appropriate therapeutic intervention, which is more effective and customized. Also, this determination at the pre-symptomatic level allows a preventive regimen to be applied.

### DRUG SCREENING

The present invention also provides novel targets and methods for the screening of drug candidates or leads. The methods include binding assays and/or functional assays, and may be performed in vitro, in cell systems, in animals, etc.

In this regard, an object of this invention resides in a method of selecting biologically active compounds on melanoma or skin cancer, the method comprising a step of selecting compounds that mimic or stimulate BCSC-1expression or activity.

A further object of this invention is a method of selecting biologically active compounds on melanoma or skin cancer, said method comprising contacting a test compound with a recombinant host cell comprising a reporter construct, said reporter construct comprising a reporter gene under the control of a BCSC-1 gene promoter, and selecting the test compounds that stimulate expression of the reporter gene.

A particular object of this invention resides in a method of selecting compounds active on melanoma or skin cancer, said method comprising contacting in vitro a test compound with a BCSC-1 gene or polypeptide and determining the ability of said test compound to bind said BCSC-1 gene or polypeptide, respectively. Binding to said gene or polypeptide provides an indication as to the ability of the compound to modulate the activity of said target, and thus to affect a pathway leading to melanoma or skin cancer. Preferably, the BCSC-1 polypeptide is a long isoform.

The determination of binding may be performed by various techniques, such as by labeling of the test compound, by competition with a labeled reference ligand, etc.

A further object of this invention resides in a method of selecting compounds active on melanoma or skin cancer, said method comprising contacting in vitro a test compound with a BCSC-1 polypeptide and determining the ability of said test compound to modulate the activity of said polypeptide. Preferably, the BCSC-1 polypeptide is a long isoform.

A further object of this invention resides in a method of selecting biologically active compounds, said method comprising contacting in vitro a test compound with a BCSC-1 gene and determining the ability of said test compound to modulate the expression of long isoform(s) of said gene.

In a particular embodiment of the methods of screening, the modulation is an activation.

The above screening assays may be performed in any suitable device, such as plates, tubes, dishes, flasks, etc. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

### PHARMACEUTICAL COMPOSITION, THERAPY

A further object of this invention is a pharmaceutical composition comprising (i) a long isoform of a BCSC-1 polypeptide, or a nucleic acid encoding the same, or a vector or a recombinant host cell as described below, and (ii) a pharmaceutically acceptable carrier or vehicle.

The invention also relates to a method of treating melanoma or skin cancer in a subject, the method comprising administering to said subject a composition as disclosed above.

The invention also relates to a method of treating melanoma or skin cancer in a subject, the method comprising administering to said subject an effective amount of a long isoform of a BCSC-1 polypeptide.

The invention also relates to the use of a BCSC-1 protein or of a nucleic acid encoding a BCSC-1 protein for the manufacture of a medicament for treating melanoma or skin cancer. Preferably, the BCSC-1 protein is a long isoform.

The invention also relates to the use of a long BCSC-1 protein isoform for the manufacture of a medicament for treating cancer.

The invention also relates to the use of a BCSC-1 agonist for the manufacture of a pharmaceutical composition for treating melanoma or skin cancer.

The invention further encompasses the use of a compound that stimulates BCSC-1 gene expression for the manufacture of a pharmaceutical composition for treating melanoma or skin cancer.

Within the context of this invention, the term "treating" includes an inhibition of tumor progression, an inhibition of tumor proliferation, a tumor size reduction, a suppression of the tumorigenicity of the tumor cells, a delay in the disease progression.

The skin cancer may be selected from basal cell carcinoma, squamous cell carcinoma, Merkel carcinoma, primary cutaneous lymphoma, as well as from any other cell proliferative disease of the skin or related surface tissues.

As shown in the experimental section, supplying BCSC-1 long isoforms to skin cancer cells suppresses tumor proliferation. The supply of such function to subjects can be accomplished through gene or protein therapy, or by administering compounds that modulate or mimic BCSC-1 polypeptide activity (e.g., agonists as identified in the above screening assays). In particular, a BCSC-1 gene may be introduced into the cells of the subject in need thereof using e.g., a viral vector or a plasmid. The gene may also be introduced as naked DNA. The gene may be provided so as to integrate into the genome of the recipient host' cells, or to remain extra-chromosomal. Integration may occur randomly or at precisely defined sites, such as through homologous recombination. Further techniques include gene gun, liposome-mediated transfection, cationic lipid-mediated transfection, etc. Gene therapy may be accomplished by direct gene injection, or by administering ex vivo prepared genetically modified cells expressing a functional BCSC-1 protein polypeptide. Protein therapy may also be accomplished, according to techniques known per se in the art. Such protein therapy is particularly suited considering that BCSC-1 is a secreted protein.

### VECTORS AND RECOMBINANT CELLS

A further aspect of this invention resides in novel products for use in diagnosis, therapy or screening.

More particularly, a further object of this invention is a vector comprising a nucleic acid encoding a BCSC-1 polypeptide, preferably a long isoform thereof. The vector may be a cloning vector or, more preferably, an expression vector, i.e., a vector comprising regulatory sequences causing expression of a BCSC-1 polypeptide from said vector in a competent host cell.

These vectors can be used to express a BCSC-1 polypeptide in vitro, ex vivo or in vivo, to create transgenic or "Knock Out" non-human animals, to amplify the nucleic acids, to express antisense RNAs, etc.

The vectors of this invention typically comprise a BCSC-1 coding sequence according to the present invention operably linked to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and may provide for ubiquitous, constitutive, regulated and/or tissue specific expression, in any appropriate host cell, including for in vivo use. Examples of promoters include bacterial promoters (T7, pTAC" Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses (e.g., lentiviruses), adenoviruses, AAVs, etc., according to recombinant DNA techniques known in the art.

The recombinant virus is preferably replication-defective, even more preferably selected from E1- and/or E4-defective adenoviruses, Gag-, pol- and/or env-defective retroviruses and Rep- and/or Cap-defective AAVs. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

A further object of the present invention resides in a recombinant host cell comprising a recombinant BCSC- gene or a vector as defined above. Suitable host cells include, without limitation, prokaryotic cells (such as bacteria) and eukaryotic cells (such as yeast cells, mammalian cells, insect cells, plant cells, etc.). Specific examples include E. coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

The present invention also relates to a method for producing a recombinant host cell expressing a BCSC-1 polypeptide according to the present invention, said method comprising (i) introducing in vitro or ex vivo into a competent host cell a recombinant nucleic acid or a vector as described above, (ii) culturing in vitro or ex vivo the recombinant host cells obtained and (iii), optionally, selecting the cells which express the BCSC-1 polypeptide.

Such recombinant host cells can be used for the production of BCSC-1 polypeptides, as well as for screening of active molecules, as described below. Such cells may also be used as a model system to study skin cancer. These cells can be maintained in suitable culture media, such as DMEM, RPMI, HAM, etc., in any appropriate culture device (plate, flask, dish, tube, pouch, etc.).

Further aspects and advantages of the present invention will be disclosed in the following experimental section, which should be regarded as illustrative and not limiting the scope of the present application.

### EXPERIMENTAL SECTION

### A- MATERIALS AND METHODS

### 1. RNA extraction and cDNA synthesis.

Total RNA was isolated from frozen skin tissue from 70 patients with different stages of melanoma and from normal naevus tissue. 1 µg of total RNA was used to synthesize cDNA using random hexamers and Supercript II reverse transcriptase (Invitrogen) following suppliers instructions.

### 2. Real-time semi-quantitative RT-PCR.

cDNA was amplified for different exons of BCSC-1. using different combinations of primers. Amplicons were designed over exon boundaries when possible, and sequences were aligned against the human genome by BLAST to ensure that they were specific for the gene being tested. The sequences of the primers used are shown in table 1. The efficiency of each design was tested with serial dilutions of cDNA. Oligonucleotides PCR reactions (10 ul volume) contained diluted cDNA, 2 x SYBR Green Master Mix (Applied Biosystems), 300 nM of forward and reverse primers. PCR were performed on a SDS 7900 HT instrument (Applied Biosystems) with the following parameters: 50°C for two minutes, 95°C for ten minutes, and 40 cycles of 95°C 15 secondes-60°C one minute. Each reaction was performed in six replicates on 384-well plate. Raw Ct values obtained with SDS 2.2 (Applied Biosystems). Four different housekeeping genes were used for the normalisation performed with GenNorm software. Relative expression was calculated as a ratio of the expression in the tumour compared with the average expression in the normal tissue (benign naevus).
Below are the oligonucleotides used in the BCSC-1 study

| Primer | Nucleotide sequence | SEQ ID NO: |
|---|---|---|
| Exon 1 - 1b S | 5'-AAATTATGACTGCAGCTGTTTTCACT-3' | 1 |
| Exon 1 - 1b AS | 5'- GTAGAACAGGCTGGGCTTCCT-3' | 2 |
| Exon 6 - 7 S | 5'- CTGATTTACTACAATGAGGTGCATACC-3' | 3 |
| Exon 6 - 7 AS | 5'- CATCAAATGACCTGGCTTCATG-3' | 4 |
| Exon 9 - 10 S | 5'- AGAAACACAGGTAGGAAGAAAATGTGA-3' | 5 |
| Exon 9 - 10 AS | 5'- TCATGTAGCACTAGAGTCTGTGTGTCA-3' | 6 |
| Exon 13 - 14 S | 5'-AGCCTGATGTCAACCTCACCAT-3' | 7 |
| Exon 13 - 14 AS | 5'- CATGTCCTTGGTCTGGAGCAA-3' | 8 |
| Exon 15 - 16 S | 5'- AGCACAGTCCAGGCTTTGGA-3' | 9 |
| Exon 15 - 16 AS | 5'- CAGGAACCATTTGCATTTTGG-3' | 10 |
| Exon 17 - 18 S | 5'- AATGGGAGCTTCTGGAAAGGA-3' | 11 |
| Exon 17 -18 AS | 5'- GCATGGTGGAGCCTGCAT-3' | 12 |

### 3. Construction and Production of Lentiviral Vectors encoding BCSC-1

The complete coding sequence of isoform F of BCSC-1 tagged with HA-protein sequence was inserted in two different lentiviral vectors (gifts from D.Trono, EPFL, Epalinges, Switzerland) and modified from (Naldini et al., 1996; Salmon et al., 2000), *PGK Ires Neo* and *PWIR GFP* plasmid. The 293T and HeLa cell lines were cultured in Dulbecco's modified Eagle's medium supplemented with 10% FCS. Recombinant lentiviruses were produced by transient transfection of 293T cells according to standard protocols. Briefly, subconfluent 293T cells were cotransfected with 20 µg of the plasmid vector (PGK BCSC Ires Neo / PWIR BCSC GFP), 15 µg of pCMV-deltaR8.91, and 5 µg of pMD2G-VSVG by calcium phosphate precipitation. After 16 h medium was changed, and recombinant lentivirus vectors were harvested 24 h later. All tests FACS, Western Blot and cell transductions were similar as previously described (Arrighi et al., 2004).

### B- EXAMPLES

### Example 1 - Identification that BCSC-1 expression is altered in skin cancers

The gene profiling technology DATAS (Differential Analysis of Transcripts with Alternative Splicing) (Schweighoffer et al., 2000) allows the isolation of a library of alternatively spliced sequences that are differentially expressed between two conditions, which library may be subsequently used for building arrays or isolating target genes.

DATAS is achieved through extraction of total RNA from 2 distinct populations (benign nevus versus metastatic melanoma) followed by messenger RNA isolation (using Dynabeads oligodT- Dynal). A reverse transcription is carried out on mRNA using biotinylated oligonucleotide dT in order to .generate first strand cDNA. Cross-hybridisations between mRNA from one population and cDNA from the other population give rise to heteroduplexes which are isolated using streptavidin-coated beads. RNAse H treatment is used to release the loops of mRNA that do not hybridise to cDNA in the heteroduplexes. These mRNA loops, which encode the alternatively spliced sequences, are subjected to RT-PCR with 5 degenerate primers followed by cloning of the PCR products in an AT cloning vector (TOPO, Invitrogen). The cDNA inserts in all the clones are amplified by PCR using the T7 primer. The final result is a library of alternatively spliced mRNA sequences unique to a given condition. After sequencing, the DATAS clones are subjected to bioinformatic analysis to identify their gene of origin. This allows the analysis of certain types of splicing events (such as exon skipping, intron retention, presence of novel exons, alternative splice site usage) with high accuracy.

We have performed DATAS profiling assays on tissue obtained from a population of healthy patients presenting benign naevi and a population of patients' suffering from metastatic melanoma. We have built a DATAS library on tissue using 11 patients from the benign naevus group and 10 patients from the metastatic melanoma group. A pool of total RNA of 80 µg was available for each population from which 1,6 µg of mRNA was extracted : 800 ng used for cDNA synthesis and 800 ng retained as mRNA. The quality and quantity of all RNA tissue samples was determined using the Agilent Bioanalyser. Only total RNA samples of good quality were used for DATAS.

A total of 755 clones resulted from DATAS on tissue RNA. Approximately 25% of the clones were obtained from the condition mRNA metastatic melanoma/cDNA benign nevus while the remaining 75% of the clones were obtained from the condition mRNA benign naevus/cDNA metastatic melanoma. The average size of the inserts was between 300 and 500 bp. After sequencing, individual clones were processed and analyzed as described above. Out of the 755 clones sequenced, 217 fragments were non redundant and contained unknown gene sequences.

From the 217 clones that we obtained from the DATAS analysis, we selected about 10 of them with potentially interesting properties and performed real-time semi-quantitative RT-PCR on RNA extracted from biopsies from melanocytic lesions. Out of this second screen, we identified BCSC-1 as a potentially interesting candidate.

### Example 2 : BCSC-1 expression is down-regulated in melanoma patients

Gene expression of BCSC-1 was determined by real-time semi-quantitative RT-PCR of RNA extracted from biopsies of a total of 70 patients, including biopsies of benign naevus (negative control) and different stages of pigmented skin lesions. The results showed that all the patients with metastatic melanoma had a significant lower expression in comparison with the mean expression of BCSC in the benign naevus samples (Figure 2). In some metastatic biopsies the expression of BCSC reach values 20 folds lower that the mean expression in benign naevus. Atypical naevus biopsies showed a higher expression of BCSC than benign naevus, and both primary melanoma and biopsies from lymph nodes, shown a similar expression of BCSC than benign naevus.

### Example 3 : Long isoforms of BCSC-1 are selectively downregulated in melanoma

Seven different isoforms have been described for the BCSC-1 gene (See Figure 3A). We performed a more specific analysis of the BCSC-1 expression using different combinations of primers to determine which isoforms were down-regulated. The results showed that longer isoforms c and f, which code for the same protein, are selectively down-regulated in melanoma patients (Figure 3B).

### Example 4 : BCSC-1 expression is down-regulated in melanoma patients and melanoma cells lines.

To confirm the previous results, new real-time PCR experiments were done in a larger cohort of biopsies from skin lesions. In agreement with the previous results, all patients with metastatic lesions had a lower expression of BCSC-1 in comparison with the mean expression in healthy donors (Figure 4A).
Moreover, several cell lines of melanocytes were tested for the expression of BCSC-1 (Figure 4B). The results showed that all melanoma cell lines have a significant lower expression than healthy donors. In some cell lines, as SK-Me123 (L1), the expression of BCSC-1 was not detectable, and only the two cell lines of primary melanoma tested show an expression of BCSC-1 more similar than benign naevus.

### Example 5 : Ectopic Expression of BCSC-1 decreases melanoma cells proliferation.

*PGK BCSC Ires Neo* or *PWIR BCSC GFP* vectors were used to transduce SK-Mel23 and Hela cells inducing the expression of BCSC-1. Moreover, cells transduced with the corresponding empty vector were used as negative control.

The results showed that ectopic expression of BCSC-1 in melanoma cell line SK-Me123 decreases markedly the proliferation of the cells (Figure 5). The expression of BCSC-1 was confirmed by Western Blot and Real-time PCR (Figure 6) using an anti-HA antibody. This effect of the ectopic expression of BCSC-1 was not observed in other non-melanocyte cell lines such as Hela.

Protein sequence of BCSC-1 (SEQ ID NO: 13)

MVHFCGLLTLHREPVPLKSISVSVNIYEFVAGVSATLNYENEEKVPLEAFFVFPMDEDSA
VYSFEALVDGKKIVAELQDKMKARTNYEKAISQGHQAFLLEGDSSSRDVFSCNVGNLQPG
SKAAVTLKYVQELPLEADGALRFVLPAVLNPRYQFSGSSKDSCLNVKTPIVPVEDLPYTL
SMVATIDSQHGIEKVQSNCPLSPTEYLGEDKTSAQVSLAAGHKFDRDVELLIYYNEVHTP
SVVLEMGMPNMKPGHLMGDPSAMVSFYPNIPEDQPSNTCGEFIFLMDRSGSMQSPMSSQD
TSQLRIQAAKETLILLLKSLPIGCYFNIYGFGSSYEACFPESVKYTQQTMEEALGRVKLM
QADLGGTEILAPLQNIYRGPSIPGHPLQLFVFTDGEVTDTFSVIKEVRINRQKHRCFSFG
IGEGTSTSLIKGIARASGGTSEFITGKDRMQSKALRTLKRSLQPVVEDVSLSWHLPPGLS
AKMLSPEQTVIFRGQRLISYAQLTGRMPAAETTGEVCLKYTLQGKTFEDKVTFPLQPKPD
VNLTIHRLAAKSLLQTKDMGLRETPASDKKDALNLSLESGVISSFTAFIAINKELNKPVQ
GPLAHRDVPRPILLGASAPLKIKCQSGFRKALHSDRPPSASQPRGELMCYKAKTFQMDDY
SLCGLISHKDQHSPGFGENHLVQLIYHQNANGSWDLNEDLAKILGMSLEEIMAAQPAELV
DSSGWATILAVIWLHSNGKDLKCEWELLERKAVAWMRAHAGSTMPSVVKAAITFLKSSVD
PAIFAF

cDNA sequence of BCSC-1 (NM_014622) – SEQ ID NO: 14

CGGTTCTTTC CGGAAATTAT GACTGCAGCT GTTTTCACTC CGCTGTGACT 50
CAGAGCGCTC CGGGCTGCAG GAGAGGAAGA AATCTTGCAT CACCATGGTG 100
CACTTCTGTG GCCTACTCAC CCTCCACCGG GAGCCAGTGC CGCTGAAGAG 150
TATCTCTGTG AGCGTGAACA TTTACGAGTT TGTGGCTGGT GTGTCTGCAA 200
CTTTGAACTA CGAGAATGAG GAGAAAGTTC CTTTGGAGGC CTTCTTTGTG 250
TTCCCCATGG ATGAAGACTC TGCTGTTTAC AGCTTTGAGG CCTTGGTGGA 300
TGGGAAGAAA ATTGTAGCAG AATTACAAGA CAAGATGAAG GCCCGCACCA 350
ACTATGAGAA AGCCATCTCC CAGGGCCACC AGGCCTTCTT ATTGGAGGGG 400
GACAGCAGCT CCAGGGATGT CTTCTCTTGC AATGTGGGTA ACCTCCAACC 450
TGGGTCGAAG GCGGCAGTCA CCCTGAAGTA TGTGCAGGAG CTGCCTCTGG 500
AAGCAGATGG GGCTCTGCGC TTTGTGCTCC CAGCTGTCCT GAATCCTAGA 550
TACCAGTTCT CTGGGTCGTC TAAGGACAGT TGCCTTAATG TGAAGACTCC 600
TATAGTCCCT GTGGAGGACC TGCCCTACAC ACTCAGCATG GTCGCCACCA 650
TAGATTCCCA GCATGGCATT GAGAAGGTCC AATCCAACTG CCCCTTGAGT 700
CCTACCGAGT ACCTAGGAGA GGACAAGACT TCTGCTCAGG TTTCCCTGGC 750
TGCTGGACAC AAGTTTGATC GGGACGTGGA ACTCCTGATT TACTACAATG 800
AGGTGCATAC CCCCAGCGTG GTTTTGGAGA TGGGGATGCC TAACATGAAG 850
CCAGGTCATT TGATGGGAGA TCCATCTGCA ATGGTGAGTT TCTATCCAAA 900
TATCCCAGAA GATCAACCAT CAAATACCTG TGGAGAGTTT ATCTTTCTCA 950
TGGACCGCTC GGGAAGTATG CAGAGCCCCA TGAGTAGCCA GGATACATCT 1000
CAGCTGCGAA TACAGGCAGC CAAGGAAACA CTGATTTTGC TGCTGAAGAG 1050
TTTACCTATA GGCTGTTATT TCAACATCTA TGGATTTGGC TCTTCCTATG 1100
AGGCATGCTT TCCGGAGAGT GTGAAGTACA CTCAGCAAAC AATGGAGGAG 1150
GCTCTGGGGA GAGTGAAGCT TATGCAGGCC GACCTAGGGG GCACTGAAAT 1200
CTTGGCACCA CTCCAGAACA TTTACAGGGG ACCCTCCATC CCAGGCCACC 1250
CCCTACAGCT TTTTGTCTTT ACAGATGGAG AAGTTACAGA CACGTTTAGT 1300
GTAATTAAAG AAGTTAGGAT CAACAGACAG AAACACAGGT GTTTCTCATT 1350
TGGTATTGGA GAAGGCACCT CCACCAGCCT AATAAAAGGT ATTGCCCGGG 1400
CATCAGGGGG CACCTCAGAA TTTATCACAG GCAAAGACAG GATGCAGTCC 1450
AAGGCTCTCA GGACTCTGAA ACGCTCTCTG CAGCCTGTGG TAGAGGATGT 1500
CTCTCTGAGC TGGCATTTGC CTCCTGGTCT GTCTGCTAAA ATGCTTTCCC 1550

CAGAACAGAC TGTCATCTTT AGGGGTCAGA GATTAATCAG CTATGCCCAG 1600
CTGACCGGGA GGATGCCAGC AGCAGAGACA ACAGGAGAAG TATGCCTCAA 1650
ATATACACTC CAGGGCAAGA CTTTTGAGGA TAAGGTGACA TTTCCTCTAC 1700
AACCCAAGCC TGATGTCAAC CTCACCATTC ACCGCCTTGC TGCCAAGTCC 1750
TTGCTCCAGA CCAAGGACAT GGGCCTCAGG GAGACTCCAG CAAGTGATAA 1800
AAAAGATGCA TTGAACCTTA GCCTTGAGTC TGGTGTCATA AGCTCCTTCA 1850
CAGCTTTCAT TGCTATCAAT AAGGAGCTCA ACAAGCCGGT TCAGGGGCCT 1900
CTGGCTCATA GGGACGTCCC AAGGCCAATT CTGTTGGGTG CTTCTGCCCC 1950
ATTGAAGATA AAATGCCAAT CAGGTTTTCG AAAGGCCTTA CACTCTGACC 2000
GTCCTCCTTC TGCATCTCAG CCCAGAGGGG AACTTATGTG TTATAAGGCC 2050
AAGACATTCC AGATGGACGA TTACAGTCTC TGTGGGTTGA TAAGTCACAA 2100
GGACCAGCAC AGTCCAGGCT TTGGAGAGAA TCACCTTGTG CAGCTGATTT 2150
ACCACCAAAA TGCAAATGGT TCCTGGGATC TGAATGAAGA TCTAGCCAAG 2200
ATCCTAGGTA TGAGTTTGGA AGAAATAATG GCTGCACAGC CTGCCGAGCT 2250
TGTGGATTCC TCAGGCTGGG CCACCATCCT GGCCGTGATC TGGCTGCACA 2300
GCAATGGTAA GGACTTGAAG TGTGAATGGG AGCTTCTGGA AAGGAAGGCC 2350
GTGGCCTGGA TGCGTGCCCA TGCAGGCTCC ACCATGCCTT CGGTTGTGAA 2400
AGCTGCTATT ACTTTCCTGA AGTCATCTGT GGATCCTGCT ATCTTTGCCT 2450
TTTGAAGATA CCATCCAGAA AAAGAAGTGC CTTTAATTTG CTACTGTCAT 2500
TTCCTCTAGT ATCACTTTTG CTGTGATGAT GTGTTCTTGT GTATTATAAC 2550
TCTTTATTTT TTGCCATAAA AGTAAAGGAT GCTTACTCCA CTTCGCTTCT 2600
CTGCTCCAGG TTCACTTTGG ATATGATCTT TCTTTTCCCA ACATATGCCC 2650
TCAGAAAAGT GACAGTGGTC CCAGAACCTA TTCCCTTTCT TGAGGGAGTT 2700
CAAAACATTC ATAGGCAGTA ATGTTCCTCC CAGGGTTTCC AGGGAAACAA 2750
CATGAAAAAC AGGTGACATG AACTACAGAC TAAAGATTGC AGCATTTATG 2800
TTAGAGAATG CTTGAATTAG AGAATTTTCT GCATTATCTT TGTCTGTTCA 2850
CTTTCTATCT TATATACTTA TCAGGGCCAT ACTGGTAAGC TTGCGTAGGA 2900
GGAGTTAGAG GGAAGTTGAA AGCCAACATC TGGATCAATG TAATGTCAAG 2950
ATCACAAAGA CAGAGACTGC AGGGGTCCAC TGTGAGAGGT GACACTGTTG 3000
GGGACCTTCC TGATTCATTC TTCTTGGGCT TTGCTAGCCT GTACAACCTA 3050
CATGTCTTTT CTTCCACTGC CTGAAAGACT TGGGTTGAAC TATAACTGTT 3100
GGAGAGAGAT GTTCCTCTTT AATCATGAAA CACCTTAAGA AGTCTATAAT 3150
GCAATCCTTA GTCCTACCCT GAACCTATGT GTCCTCTAAG TCAGGCCCTG 3200
ATCTAGTGCA GTAAAGGGAA GGGTGGGCTT AATGGGAGCT TTGCCTGGGA 3250
CCTGAACCTG GAGCACTTAC CGCATTAGGA AGAAAGGAGC TCCCCGTAAT 3300
CGTTCCTGAC CCTTGTGTCT CATATACCCT ATCCTGGTGG AAATGACCCT 3350
ATTTGATATG CTGTCCCTTA AAATAACTTG TATCAATATT AAAATGACTA 3400
TTTCTACCCT TTGATGAGTa aaaaaaaaaa aaaaaaaa

### References

Arrighi, J. F., Pion, M., Wiznerowicz, M., Geijtenbeek, T. B., Garcia, E., Abraham, S., Leuba, F., Dutoit, V., Ducrey-Rundquist, O., Van Kooyk, Y., et al. (2004). Lentivirus-Mediated RNA Interference of DC-SIGN Expression Inhibits Human Immunodeficiency Virus Transmission from Dendritic Cells to T Cells. J Virol 78, 10848-10855. Naldini, L., Blomer, U., Gallay, P., Ory, D., Mulligan, R., Gage, F. H., Verma, I.M., and Trono, D. (1996). In vivo gene delivery and stable transduction of nondividing cells by a lentiviral vector. Science 272, 263-267. Salmon, P., Kindler, V., Ducrey, O., Chapuis, B., Zubler, R. H., and Trono, D. (2000). High-level transgene expression in human hematopoietic progenitors and differentiated blood lineages after transduction with improved lentiviral vectors. Blood 96, 3392-3398. Schweighoffer, F., Ait-lkhlef, A., Resink, A. L., Brinkman, B., Melle-Milovanovic, D., Laurent-Puig, P., Kearsey, J., and Bracco, L. (2000). Qualitative gene profiling: a novel tool in genomics and in pharmacogenomics that deciphers messenger RNA isoforms diversity. Pharmacogenomics 1, 187-197.

## Claims

1. A method for detecting the presence, stage or type of a melanoma or skin cancer in a subject, the method comprising detecting in vitro or ex vivo the presence of an altered BCSC-1 gene expression in a sample from the subject, the presence of such an altered BCSC-1 gene expression being indicative of the presence, stage or type of melanoma or skin cancer in said subject.

2. The method of claim 1, wherein a reduced expression of a BCSC-1 gene in said sample is indicative of the presence, stage or type of melanoma or skin cancer in said subject.

3. The method of claim 1 or 2, wherein a reduced expression of a long isoform of a BCSC-1 gene in said sample is indicative of the presence, stage or type of melanoma or skin cancer in said subject.

4. The method of claim 3, wherein the long isoform comprises exon 13, 14, 15, 16, 17 or 18.

5. A method of assessing the efficacy of a treatment of melanoma or skin cancer in a subject, the method comprising comparing BCSC-1 gene expression in a sample from the subject prior to and after said treatment, an increased expression being indicative of a positive response to treatment.

6. A method of any one of claims 1-5, wherein the BCSC-1 gene expression is detected by sequencing, selective hybridization, selective amplification and/or specific ligand binding.

7. The method of any one of claims 1 to 6, wherein the cancer is selected from basal cell carcinoma, squamous cell carcinoma, Merckel carcinoma and melanoma.

8. A nucleic acid primer that allows amplification of a long isoform of BCSC-1.

9. A nucleic acid probe that specifically hybridizes to a long isoform of BCSC-1.

10. An antibody that specifically binds a long isoform of a BCSC-1 protein.

11. A kit comprising a primer, probe or antibody according to any one of claims 8 to 10, respectively.

12. The use of a BCSC-1 protein or of a nucleic acid encoding a BCSC-1 protein for the manufacture of a medicament for treating melanoma or skin cancer.

13. The use of a BCSC-1 agonist for the manufacture of a pharmaceutical composition for treating melanoma or skin cancer.

14. The use of a compound that stimulates BCSC-1 gene expression for the manufacture of a pharmaceutical composition for treating melanoma or skin cancer.

15. A method of selecting biologically active compounds on melanoma or skin cancer, the method comprising a step of selecting compounds that mimic or stimulate BCSC-1 expression or activity.

16. A method of selecting biologically active compounds on melanoma or skin cancer, said method comprising contacting a test compound with a recombinant host cell comprising a reporter construct, said reporter construct comprising a reporter gene under the control of a BCSC-1 gene promoter, and selecting the test compounds that stimulate expression of the reporter gene.

17. A method for detecting the presence, stage or type of a cancer in a subject, the method comprising determining in vitro or ex vivo the (relative) amount of a long isoform of BCSC-1 gene or protein in a sample from the subject, such amount being indicative of the presence, stage or type of a cancer in said subject.

18. A method for detecting the presence, stage or type of a cancer in a subject, the method comprising determining in vitro or ex vivo the (relative) amount of BCSC-1 protein in a fluid sample derived from the subject, such amount being indicative of the presence, stage or type of a cancer in said subject.
